# FASCICULE DE BREVET EUROPEEN

(11) **EP 0 574 511 B1**
(45) Date de publication et mention de la délivrance du brevet: **05.10.1994**
(21) Numéro de dépôt: 92907936.6
(22) Date de dépôt: 04.03.1992
(51) Int. Cl.: G01N 25/48, B01J 8/06, C07H 3/06, C07H 21/00, C07K 1/04

(54) **PROCEDE ET APPAREIL DE SYNTHESE EN PHASE HETEROGENE DE MACROMOLECULES TELLES QUE DES PEPTIDES, DES POLYNUCLEOTIDES OU DES OLIGOSACCHARIDES**
Verfahren und Vorrichtung zur Herstellung in heterogener Phase von Makromolekülen wie Peptiden, Polynuklotiden oder Polysocchariden
METHOD AND DEVICE FOR HETEROGENEOUS PHASE SYNTHESIS OF MACROMOLECULES SUCH AS PEPTIDES, POLYNUCLEOTIDES OR OLIGOSACCHARIDES

(30) Priorité: 06.03.1991 FR 9102645
(43) Date de publication de la demande: 22.12.1993
(73) Titulaire: RHONE-POULENC RORER S.A., 92160 Antony (FR)
(72) Inventeur: LAPLUYE, Gérard, F-78000 Versailles (FR); POISSON, Roger, F-78340 Les-Clayes-sous-Bois (FR)
(74) Mandataire: Ricalens, François
(86) Numéro de dépôt international: FR9200197
(87) Numéro de publication internationale: WO9215867

(56) Documents cités:
- WO-A-82/03077
- DE-A- 3 536 349
- DE-A- 3 723 004
- REVIEW OF SCIENTIFIC INSTRUMENTS, Vol. 44, No. 9, 1 September 1973, NEW YORK US, pages 1234 - 1238; S. AMDUR et al.: "An improved adiabatic titration calorimeter"

## Description

L'invention concerne un procédé et un appareil de synthèse en phase hétérogène de molécules et macromolécules, telles que des peptides, des oligo- ou polynucléotides ou des oligo- ou poly-saccharides.

La synthèse de ces molécules sur support solide, et notamment celle des peptides, est bien connue depuis les travaux de MERRIFIELD en 1963, et est largement répandue. Elle consiste essentiellement à assembler séquentiellement des acides aminés pour former un peptide, en fixant une extrémité de la chaîne sur un support insoluble, tel qu'un polymère. Lorsque la séquence désirée d'acides aminés a été assemblée séquentiellement, on réalise un clivage de cette chaîne pour séparer le peptide du support et le libérer en solution.

Les réactions mises en jeu dans cette synthèse sont essentiellement des réactions de couplage des acides aminés, réalisées en alternance avec des réactions de déprotection des fonctions voulues des acides aminés, et des opérations de lavage et de rinçage entre la fin d'une réaction et le début de la réaction suivante.

Il est essentiel, dans cette synthèse, que chaque réaction de couplage ou de déprotection soit réalisée à 100% ou presque. Le seul moyen permettant actuellement de s'en assurer est d'interrompre la synthèse pour réaliser un test ou un dosage. Cela allonge considérablement la durée d'une synthèse et interdit son automatisation. De plus, les tests utilisés ne permettent pas toujours de vérifier qu'une réaction a eu lieu complètement, de sorte que l'on est conduit, en cas d'incertitude, à recommencer cette réaction pour plus de sécurité.

Enfin, on est en général obligé, dans ces procédés classiques de synthèse, d'utiliser des excès de constituants et de réactifs de couplage, pour de multiples raisons (inaccessibilité d'une partie des sites réactionnels du polymère, faible accrochage des constituants sur le polymère ou sur un constituant déjà fixé sur le polymère, etc).

Tous ces inconvénients font que les procédés classiques de synthèse des peptides ont un rendement faible, de l'ordre de 20 à 30% en fonction de la longueur de la chaîne du peptide, et que le coût des peptides est extrêmement élevé (parfois plus de 1000 FRF/milligramme de peptide).

Pour tenter de réduire ces inconvénients, on a déjà proposé de suivre les réactions de couplage par spectroscopie ultra-violette, censée détecter un pic de signal correspondant à la fin de la réaction de couplage. Les résultats ont montré cependant que cette méthode n'était pas très fiable et pouvait conduire à des erreurs ou à de grandes difficultés d'interprétation.

Le document WO 82/03077 divulge un procédé de synthèse de macromolécules où la première réaction de couplage est controlée par spéctrophotometrie ou par analyse quantitative d'acide aminé.

Il a également été proposé de mesurer la conductance électrique du milieu réactionnel. Cependant, ce milieu est particulièrement hétérogène et on détecte dans ce procédé un bruit de fond très important, dans lequel le signal utile est noyé, ce qui le rend difficilement exploitable.

L'invention a essentiellement pour but d'apporter une solution simple, efficace et particulièrement fiable au problème du suivi des réactions de couplage et de déprotection réalisées dans les synthèses de peptides, de polynucléotides ou d'oligosaccharides.

Elle est basée sur le fait que chaque réaction de couplage ou de déprotection provoque une variation de température du milieu réactionnel et que, dans certaines conditions, on peut considérer que la réaction est terminée lorsque cette variation de température devient sensiblement nulle.

Il est cependant à peu près impossible de mesurer directement cette variation de température, car elle est beaucoup plus faible (d'un ou de plusieurs ordres de grandeur) que les variations de température dues à des réactions parasites dans le milieu réactionnel, notamment des réactions entre solvants.

L'un des mérites essentiels de la présente invention est donc de réussir à détecter la très faible variation de température du milieu réactionnel, qui accompagne une réaction de couplage ou de déprotection, et de suivre de façon très précise l'évolution de cette variation de température pour détecter de façon sûre et fiable la fin de la réaction de couplage ou de déprotection.

L'invention propose donc un procédé de synthèse en phase hétérogène de macromolécules telles que des peptides, des polynucléotides ou des oligosaccharides, consistant à disposer un support solide tel qu'un polymère dans un réacteur tubulaire, à doser et à amener séquentiellement dans le réacteur des liquides réactifs pour réaliser successivement les réactions voulues, notamment de couplage et de déprotection, en alternance avec des opérations de lavage et de rinçage du réacteur et du support solide, le procédé étant caractérisé en ce qu'il consiste à fermer les extrémités supérieure et inférieure du réacteur par des filtres retenant le support solide et laissant passer les liquides, puis, pour chaque réaction à réaliser, à faire circuler le liquide réactif correspondant de façon répétée et alternative dans le réacteur et à travers les filtres précités, et à suivre l'évolution de la réaction en cours par détection des variations de température du liquide réactif liées à ses passages dans le réacteur et hors du réacteur, la fin de la réaction correspondant à une valeur sensiblement nulle de ces variations de température après une décroissance progressive durant un intervalle de temps prédéterminé.

La circulation répétée et alternative du liquide réactif dans le réacteur, permet de réaliser une sorte de modulation de la variation de température de ce liquide réactif qui est directement due à une réaction de couplage ou de déprotection en cours, et donc de mettre en évidence cette faible variation de température par rapport à une variation globale beaucoup plus importante de la température du liquide réactif, due à des réactions parasites d'estérification d'acides aminés, d'interaction entre groupements, et essentiellement à des mélanges de solvants.

De préférence, la circulation du liquide réactif dans le réacteur et hors de celui-ci est périodique et verticale.

En choisissant de façon appropriée la fréquence de cette circulation périodique alternative, on peut facilement mettre en évidence la modulation de la variation de température due à une réaction de couplage ou de déprotection, la fréquence de cette modulation correspondant à celle de la circulation périodique alternative du liquide.

Dans un mode de réalisation préféré de l'invention, le procédé consiste à déplacer le réacteur en mouvement rectiligne alternatif vertical dans une enceinte contenant le liquide réactionnel.

Selon encore une autre caractéristique de l'invention, le procédé consiste à détecter de façon continue la température du liquide réactif en un point situé au voisinage immédiat d'un filtre du réacteur, le point de détection de la température étant lié au réacteur pour suivre ce dernier dans son mouvement alternatif, et étant avantageusement situé à l'extérieur du réacteur, dans un passage de guidage du liquide réactif vers l'extrémité correspondante du réacteur.

On améliore ainsi la sensibilité de la détection des variations de température du liquide réactif.

L'invention propose également un appareil de synthèse en phase hétérogène de macromolécules telles que des peptides, des polynucléotides, ou des oligosaccharides, cet appareil comprenant un réacteur tubulaire contenant un support solide tel qu'un polymère, et des moyens de dosage, d'amenée et d'évacuation séquentiels de liquides réactifs et de liquides de lavage et de rinçage du réacteur et du support solide, caractérisé en ce que le réacteur tubulaire est fermé à ses extrémités par des filtres retenant le support solide et laissant passer les liquides, et en ce que l'appareil comprend des moyens pour faire circuler de façon répétée et alternative le liquide réactif dans le réacteur et à travers les filtres précités, et des moyens de détection des variations de température du liquide réactif liées à ses passages dans le réacteur et hors du réacteur.

De préférence, le réacteur est monté mobile en mouvement alternatif rectiligne vertical dans une enceinte comprenant des moyens d'amenée et d'évacuation des liquides précités, et est relié à des moyens moteurs de déplacement.

Le réacteur est monté coulissant à étanchéité dans cette enceinte et comporte à ses extrémités des joints d'étanchéité coopérant avec une paroi cylindrique de l'enceinte et emprisonnant entre eux une couche de gaz, par exemple d'air, formant isolation thermique du réacteur.

Les moyens précités de détection de température comprennent au moins une sonde de température, telle qu'une thermistance, placée au contact du liquide réactif en un point situé au voisinage d'un des filtres d'extrémité du réacteur.

Cette sonde de température est avantageusement reliée à une entrée d'un système de traitement de l'information dont les sorties sont reliées à des moyens de commande de dosage, d'amenée et d'évacuation des liquides précités, ainsi qu'éventuellement aux moyens moteurs de déplacement du réacteur dans ladite enceinte.

Des moyens d'enregistrement et/ou de visualisation du signal de sortie de la sonde de température peuvent également être prévus.

L'invention sera mieux comprise et d'autres caractéristiques, détails et avantages de celle-ci apparaîtront plus clairement à la lecture de la description qui suit, faite à titre d'exemple en référence aux dessins annexés dans lesquels :
la figure 1 représente schématiquement un appareil selon l'invention;
les figures 2, 3 et 4 représentent schématiquement des variations de température d'un liquide réactif dues à des réactions de couplage et de déprotection, respectivement.

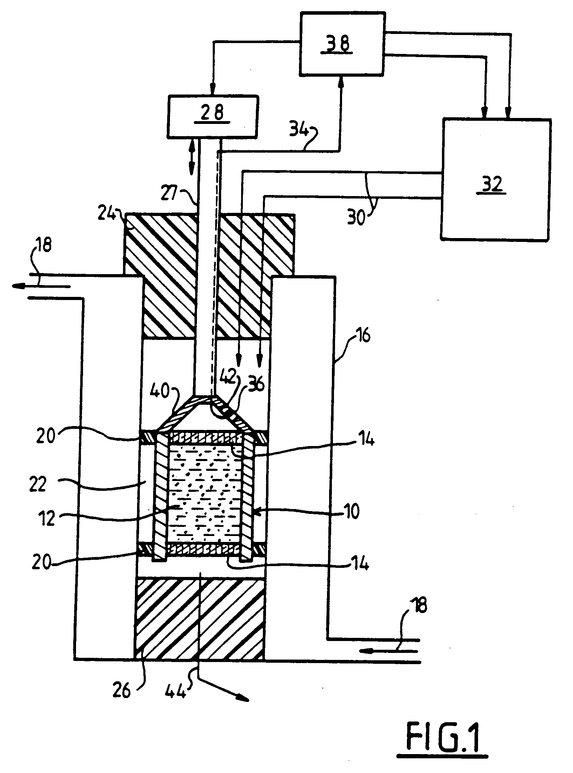

En figure 1, on a représenté schématiquement un appareil selon l'invention, utilisable notamment pour la synthèse de peptides, de polynucléotides ou d'oligosaccharides.

Cet appareil comprend essentiellement un réacteur tubulaire 10, par exemple de forme cylindrique, destiné à contenir un support solide 12 tel qu'une résine par exemple, et dont les extrémités axiales sont fermées par des filtres 14 permettant de retenir cette résine tout en laissant passer les liquides réactifs et des liquides de lavage et de rinçage.

Typiquement, les filtres 14 sont constitués de disques de matière poreuse ou microporeuse insensible aux solvants et aux agents intervenant dans les réactions à réaliser. On peut par exemple utiliser des disques microporeux en verre fritté, en métal, en polytétrafluoréthylène, en élastomère fluoré, etc...

Dans le mode de réalisation représenté, le réacteur 10 est monté coulissant verticalement à étanchéité dans une enceinte cylindrique 16 à double paroi, qui est maintenue à température constante, par exemple par circulation d'un liquide thermorégulé, dans le sens indiqué par les flèches 18.

Le réacteur 10 comporte, à ses extrémités, des joints d'étanchéité 20, par exemple en polytétrafluoréthylène, qui coopèrent avec la paroi cylindrique interne de l'enceinte 16 et qui emprisonnent entre eux une couche annulaire 22 de gaz, par exemple d'air, qui forme une isolation thermique du réservoir 10.

Les extrémités supérieure et inférieure de l'enceinte 16 sont fermées par des bouchons 24 et 26 respectivement, par exemple en polytétrafluoréthylène, qui comportent des moyens d'amenée et d'évacuation des liquides réactifs et des liquides de lavage et de rinçage.

Plus précisément, le bouchon supérieur 24 de l'enceinte 16 est traversé, d'une part, par une tige rectiligne 27 reliant le réacteur 10 à des moyens moteurs 28 de déplacement du réacteur en mouvement rectiligne alternatif dans l'enceinte 16, et, d'autre part, par des tubes 30 d'amenée des liquides précités, qui sont raccordés, en amont, à des moyens automatiques 32 de dosage et de distribution.

La tige 27 précitée peut être tubulaire, de façon à recevoir les conducteurs 34 reliant une sonde de température 36 à une entrée d'un système 38 de traitement de l'information, comprenant des moyens d'enregistrement et/ou d'affichage d'un signal de température.

La sonde de température 36, telle qu'un thermocouple, une thermistance, ou tout autre moyen approprié, est située au voisinage immédiat d'un filtre 14 du réacteur 10, à l'extérieur de ce réacteur et à l'intérieur d'un passage de liquide, ce passage étant par exemple formé d'un couvercle tronconique 40 coiffant l'extrémité supérieure du réacteur 10 et comportant un orifice 42 dans lequel est logée la sonde de température 36.

Le système 38 de traitement de l'information est avantageusement programmé pour la commande des moyens moteurs 28 et des moyens 32 de dosage et de distribution des liquides.

Le bouchon inférieur 26 de l'enceinte 16 comporte quant à lui un conduit 44 d'évacuation des liquides.

L'appareil selon l'invention est utilisé par exemple de la façon suivante :
on commence par placer dans le réacteur 10 une quantité de polymère de support (par exemple des microbilles de résine) déterminée en fonction du volume de ce réacteur et de la quantité de peptide, de polynucléotide ou d'oligosaccharide à synthétiser.

Le réacteur 10 muni de ses filtres 14 est ensuite placé à l'intérieur de l'enceinte 16, en étant relié aux moyens d'entraînement 28.

Une première étape du procédé consiste à neutraliser le polymère de support, par rinçage au D.I.E.A. (Diisopropyléthylamine) dilué dans du D.C.M (Dichlorométhane) puis par rinçage au D.C.M.

Les poudres des réactifs [HOBT (1-hydroxybenzotriazole), BOP (Benzotriazoly-N-oxy-tris-(diméthylamino)-phosphonium-hexafluorophosphate), t-BOC-A.A (tertiobutyl-oxycarbonyl-amino-acide)] sont en attente dans un récipient. On prélève un mélange de D.C.M. et de D.M.F (Dimétylformamide) qu'on introduit dans le récipient contenant les poudres. Celles-ci sont dissoutes, puis le mélange liquide est ensuite introduit dans le réacteur 10.

Simultanément, une quantité de D.I.E.A. est ajoutée au mélange dans le réacteur, et ce dernier est déplacé en mouvement alternatif rectiligne vertical dans l'enceinte 16 par les moyens moteurs 28. Ce mouvement alternatif est continu, sa fréquence étant par exemple de deux aller-retours par minute pour une réaction de couplage, et de sept à dix aller-retours par minute pour un rinçage. La course de déplacement du réacteur 10 dans un sens ou dans l'autre est déterminée de préférence de façon à ce que le volume de liquide pénétrant dans le réacteur par une extrémité et sortant du réacteur par l'autre extrémité, soit sensiblement égal au volume laissé libre dans le réacteur par le polymère de support.

Ce mouvement alternatif rectiligne du réacteur dans l'enceinte 16 se traduit, non seulement par une circulation alternative périodique du liquide réactif à l'intérieur du réacteur, mais également par un brassage et une mise en suspension du polymère de support à l'intérieur du réacteur. Par exemple, si le polymère de support est plus dense que le liquide réactif, le mouvement vers le haut du réacteur se traduit par une sorte de tassement du polymère de support au fond du réacteur, tandis que son mouvement vers le bas se traduit par une remise en suspension du polymère de support dans le réacteur. De plus, le mouvement alternatif du réacteur a pour effet de décolmater les pores des filtres 14, qui seraient autrement susceptibles d'être progressivement bouchés par le polymère de support.

La sonde 36 permet de suivre l'évolution de la réaction en cours (réaction de couplage ou réaction de déprotection d'une fonction d'un constituant).

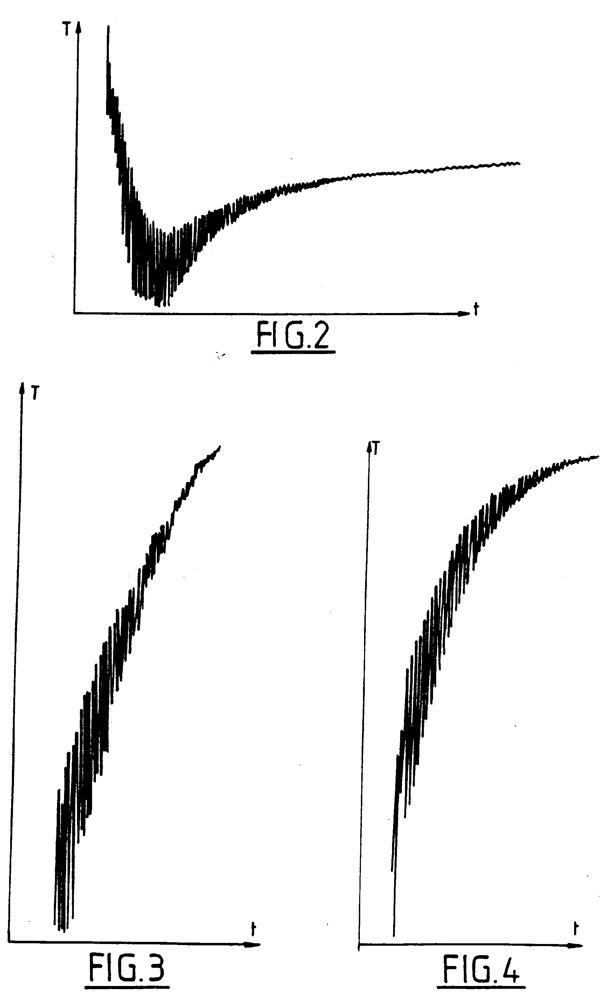

On a représenté en figure 2 la courbe de variation du signal de sortie de la sonde 36 en fonction du temps, au cours d'une réaction de couplage, qui dans ce cas précis était une réaction de couplage Asn-Gly (Asparagine-Glycocolle). On voit que cette courbe comporte une oscillation en dents de scie autour d'une valeur moyenne qui décroît assez rapidement, puis croît à nouveau jusqu'à une valeur sensiblement constante, et que l'amplitude de l'oscillation en dents de scie diminue progressivement au cours du temps pour devenir sensiblement nulle.

Cette courbe s'interprète de la façon suivante:
les dents de scie représentent les variations de température du liquide réactif dues aux aller-retours du réacteur 10 dans l'enceinte 16. Lorsque le réacteur descend dans l'enceinte à partir de son point mort haut, le liquide présent dans le réacteur passe à travers le filtre supérieur 14, remplit le couvercle tronconique 40 et sort par l'orifice 42 de celui-ci pour venir en partie supérieure de l'enceinte. Le signal fourni par la sonde 36 correspond alors à la température du liquide soumis à la réaction de couplage. Inversement, lorsque le réacteur 10 remonte dans l'enceinte 16 à partir de son point mort bas, le liquide contenu dans le réacteur 10 sort de celui-ci en traversant son filtre inférieur 14, et le liquide contenu dans la partie supérieure de l'enceinte 10 au-dessus du réacteur, pénètre dans le couvercle tronconique 40 en passant par l'orifice 42, puis rentre dans le réacteur 10 en traversant le filtre supérieur 14.

La sonde de température 36 est donc en présence successivement du liquide réactif qui vient d'être soumis à une réaction de couplage, puis du liquide réactif qui va participer une nouvelle fois à la réaction de couplage.

Lorsque le signal de sortie de la sonde 36 ne présente plus d'oscillation en dents de scie correspondant au mouvement du réacteur, cela signifie que la réaction de couplage est terminée.

L'expérience a permis de vérifier, au moyen de tests et de dosages, qu'il en était ainsi pour toutes les réactions de couplage et toutes les réactions de déprotection utilisées.

La figure 3 représente les variations du signal de sortie de la sonde 36 lors d'une réaction de déprotection (libération d'une fonction α-NH2 au T.F.A (acide trifluoroacétique)).

La figure 4 représente les variations de ce signal de sortie lors d'un couplage Gly-résine.

On voit sur les courbes des figures 2, 3 et 4 que la valeur moyenne du signal de sortie de la sonde de détection de température peut varier de façon très importante par rapport à l'amplitude de l'oscillation en dents de scie, cette variation de la valeur moyenne étant due essentiellement à des réactions secondaires d'estérification, à des interactions entre groupements des constituants, et surtout à des dégagements de chaleur provoqués par des mélanges de solvants. Les amplitudes de l'oscillation en dents de scie peuvent être de l'ordre du 1/10 de degré C, alors que la valeur moyenne du signal de sortie de la sonde peut varier de plusieurs degrés C au cours d'une réaction. Il n'est toutefois nécessaire de connaître ni la valeur moyenne du signal de sortie de la sonde thermique, ni l'amplitude de son oscillation en dents de scie. Il suffit, pour vérifier qu'une réaction de couplage ou de déprotection se déroule normalement et se termine, de constater que l'amplitude de l'oscillation en dents de scie du signal de la sonde thermique diminue progressivement et finit par s'annuler au bout d'un intervalle de temps dont l'ordre de grandeur est connu (de 10 à 30 minutes en général, selon le type de réaction).

Ce signal de sortie de la sonde thermique est donc analysable sans difficulté par un système de traitement de l'information d'un type classique, qui, en fin de réaction, peut commander le passage à la phase suivante du procédé ou qui, si une réaction ne se déroule pas correctement, peut détecter et signaler une anomalie de fonctionnement (par exemple blocage d'une vanne, mauvais fonctionnement d'un doseur, etc).

L'invention permet donc de suivre automatiquement le déroulement des réactions de couplage et de déprotection, et de vérifier, sans prélèvement, ni interruption du processus, qu'une réaction s'est correctement déroulée et est terminée, avant de passer à la phase suivante du processus.

De façon générale, l'invention permet de synthétiser des peptides avec un rendement supérieur à 95% en sortie de clivage, avant toute purification, tout en utilisant environ 2 fois moins d'acide aminé par équivalent de support que dans la technique antérieure.

Par exemple, un décapeptide A.C.P 63-74 (correspondant aux acides aminés 63 à 74 de l'acyl carrier protein) a été synthétisé en 15 heures de fonctionnement de l'appareil, réparties sur deux jours. La température de l'enceinte était régulée à 25°C. On a pu obtenir, après clivage, 350 mg de peptide pour 1 g de peptidyl-résine de départ, 96% de la masse de ce peptide étant composé du décapeptide recherché.

De même, un tétrapeptide Val-Tyr-Gly-Gly a été synthétisé avec un degré de pureté de 97,3% après clivage, et un tripeptide Gly-Val-Ala a été synthétisé avec un degré de pureté de 98,5% après clivage.

L'invention s'applique également aux synthèses de polynucléotides et d'oligosaccharides, et permet de la même façon que décrite ci-dessus, de suivre les réactions mises en jeu dans ces synthèses.

Par ailleurs, de nombreuses modifications peuvent être apportées au mode de réalisation. On peut par exemple utiliser deux sondes de température, l'une placée à l'intérieur du réacteur et l'autre à l'extérieur, et surveiller la variation de la différence de leurs signaux de sortie.

On peut également, au lieu de déplacer le réacteur en mouvement alternatif rectiligne dans une enceinte, le laisser fixe et faire circuler le liquide réactif de façon alternative dans le réacteur.

## Revendications

1. Procédé de synthèse en phase hétérogène de macromolécules telles que des peptides, des polynucléotides ou des oligosaccharides, consistant à déposer un support solide tel qu'un polymère dans un réacteur tubulaire, à doser et à amener séquentiellement dans le réacteur des liquides réactifs pour réaliser successivement des réactions voulues de couplage et de déprotection, en alternance avec des opérations de lavage et de rinçage du réacteur et du support solide, caractérisé en ce qu'il consiste à fermer les extrémités supérieure et inférieure du réacteur (10) par des filtres (14) retenant le support solide et laissant passer les liquides, puis, pour chaque réaction à réaliser, à faire circuler le liquide réactif correspondant de façon répétée et alternative dans le réacteur (10) et à travers les filtres (14) précités, et à suivre l'évolution de la réaction en cours par détection des variations de température du liquide réactif liées à ses passages dans le réacteur et hors du réacteur, la fin de la réaction correspondant à une valeur sensiblement nulle de ces variations de température après une décroissance progressive durant un intervalle de temps prédéterminé.

2. Procédé selon la revendication 1, caractérisé en ce que la circulation alternative du liquide dans le réacteur (10) est périodique.

3. Procédé selon la revendication 1 ou 2, caractérisé en ce que la circulation alternative du liquide dans le réacteur (10) est verticale.

4. Procédé selon la revendication 1, 2 ou 3, caractérisé en ce qu'il consiste à déplacer le réacteur (10) en mouvement rectiligne alternatif vertical dans une enceinte (16) contenant le liquide réactif.

5. Procédé selon une des revendications 2 à 4, caractérisé en ce qu'il consiste à détecter de façon continue la température du liquide réactif en un point situé au voisinage immédiat d'un filtre (14) du réacteur.

6. Procédé selon l'ensemble des revendications 4 et 5, caractérisé en ce que le point de détection de la température est lié au réacteur (10) et suit ce dernier dans son mouvement alternatif.

7. Procédé selon la revendication 5 ou 6, caractérisé en ce que le point de détection de température est situé à l'extérieur du réacteur (10) dans un passage (40, 42) de guidage du liquide réactif vers l'extrémité correspondante du réacteur.

8. Procédé selon l'une des revendications 4 à 7, caractérisé en ce que le volume de liquide réactif circulant dans le réacteur lors de chaque déplacement de celui-ci dans un sens et dans l'autre est sensiblement égal au volume laissé libre dans le réacteur par le polymère de support.

9. Procédé selon l'une des revendications précédentes, caractérisé en ce que le réacteur (10) est thermiquement isolé.

10. Appareil de synthèse en phase hétérogène de macromolécules telles que des peptides, des polynucléotides ou des oligosaccharides, comprenant un réacteur tubulaire (10) contenant un support solide (12) tel qu'un polymère, et des moyens (30, 32, 44) de dosage, d'amenée et d'évacuation séquentiels de liquides réactifs et de liquides de lavage et de rinçage du réacteur et du support solide, caractérisé en ce que le réacteur tubulaire (10) est fermé à ses extrémités par des filtres (14) retenant le support solide et laissant passer les liquides, et en ce que l'appareil comprend des moyens pour faire circuler de façon répétée alternative le liquide réactif dans le réacteur et à travers les filtres précités, et des moyens (36) de détection des variations de température du liquide réactif liées à ses passages dans le réacteur et hors du réacteur.

11. Appareil selon la revendication 10, caractérisé en ce que la circulation du liquide réactif dans le réacteur (10) est périodique.

12. Appareil selon la revendication 10 ou 11, caractérisé en ce que le réacteur (10) est monté mobile en mouvement alternatif rectiligne vertical dans une enceinte (16) comprenant des moyens d'amenée et d'évacuation du liquide précité, et est relié à des moyens moteurs (28) de déplacement.

13. Appareil selon la revendication 12, caractérisé en ce que le réacteur (10) est monté coulissant à étanchéité dans l'enceinte (16).

14. Appareil selon la revendication 12 ou 13, caractérisé en ce que le réacteur comporte à ses extrémités des joints d'étanchéité (20) coopérant avec une paroi cylindrique de l'enceinte (16) et emprisonnant entre eux une couche de gaz, par exemple d'air, formant isolation thermique.

15. Appareil selon l'une des revendications 12 à 14, caractérisé en ce que l'enceinte (16) est maintenue à température constante, par exemple par circulation d'un liquide thermorégulé.

16. Appareil selon l'une des revendications 10 à 15, caractérisé en ce que les moyens de détection de température comprennent au moins une sonde de température, telle qu'une thermistance par exemple, placée au contact du liquide réactif en un point situé au voisinage d'un des filtres (14) précités.

17. Appareil selon la revendication 16, caractérisé en ce que la sonde de température (36) est à l'extérieur du réacteur et est liée à ce dernier.

18. Appareil selon la revendication 16 ou 17, caractérisé en ce que la sonde de température (36) est placée dans un passage (40, 42) canalisant le liquide réactif vers l'extrémité correspondante du réacteur.

19. Appareil selon l'une des revendications 16 à 18, caractérisé en ce que ladite sonde de température (36) est reliée à une entrée d'un système (38) de traitement de l'information, dont les sorties sont reliées à des moyens (32) de commande de dosage, d'amenée et d'évacuation des liquides réactifs.

20. Appareil selon la revendication 19, caractérisé en ce que son fonctionnement et la succession des réactions de couplage et de déprotection sont commandés par le système (38) de traitement de l'information.

## Patentansprüche

1. Verfahren zur Synthese von Makromolekülen wie Peptiden, Polynucleotiden oder Oligosacchariden in heterogener Phase, das darin besteht, einen festen Träger wie ein Polymer in einen röhrenlörmigem Reaktor einzubringen, die Reaktionsflüssigkeiten zu dosieren und nacheinander zuzuführen, um aufeinanderfolgend erwünschte Kupplungsreaktionen und Reaktionen zur Entfernung von Schutzgruppen durchzuführen, abgewechselt von Wasch- und Spülvorgängen des Reaktors und des festen Trägers, dadurch gekennzeichnet, daß es darin besteht, den obersten und untersten Teil des Reaktors (10) mit Filtern (14) zu verschließen, die den festen Träger zurückhalten und die Flüssigkeiten hindurchlaufen lassen, dann die entsprechende Reaktionsflüssigkeit für jede durchzuführende Reaktion wiederholt und in abwechselnder Richtung in dem Reaktor (10) und durch die vorgenannten Filter (14) in Umlauf zu bringen, und darin, die Entwicklung der laufenden Reaktion durch Detektion der Temperaturschwankungen der Reaktionsflüssigkeit zu verfolgen, die mit ihren Durchläufen durch den Reaktor und außerhalb des Reaktors verbunden sind, wobei das Ende der Reaktion einem Wert dieser Temperaturschwankungen von praktisch Null entspricht nach einer fortschreitenden Abnahme während eines vorher festgelegten Zeitintervalls.

2. Verfahren gemäß Anspruch 1, dadurch gekennzeichnet, daß die Strömung der Flüssigkeit in abwechselnder Richtung durch den Reaktor (10) periodisch erfolgt.

3. Verfahren gemäß Anspruch 1 oder 2, dadurch gekennzeichnet, daß die Strömung der Flüssigkeit in abwechselnder Richtung durch den Reaktor (10) vertikal verläuft.

4. Verfahren gemäß Anspruch 1, 2 oder 3, dadurch gekennzeichnet, daß es darin besteht, den Reaktor (10) in einer geradlinigen, die Richtung wechselnden, vertikalen Bewegung in einen umschlossenen Raum (16) zu verschieben, der die Reaktionsnüssigkeit enthält.

5. Verfahren gemäß einem der Ansprüche 2 bis 4, dadurch gekennzeichnet, daß es darin besteht, die Temperatur der Reaktionsflüssigkeit kontinuierlich zu detektieren, und zwar an einem Punkt, der sich in unmittelbarer Nähe eines Filters (14) des Reaktors befindet.

6. Verfahren gemäß der Gesamtheit der Ansprüche 4 und 5, dadurch gekennzeichnet, daß der Detektionspunkt für die Temperatur mit dem Reaktor (10) verbunden ist und letzterem in seiner die Richtung wechselnden Bewegung folgt.

7. Verfahren gemäß Anspruch 5 oder 6, dadurch gekennzeichnet, daß der Detektionspunkt für die Temperatur außerhalb des Reaktors (10) liegt in einem Durchgang (40, 42) zur Führung der Reaktionsflüssigkeit zum entsprechenden äußersten Ende des Reaktors.

8. Verfahren gemäß einem der Ansprüche 4 bis 7, dadurch gekennzeichnet, daß das Volumen der Reaktionsflüssigkeit, das sich in dem Reaktor bei jeder Verschiebung desselben in die eine oder die andere Richtung im Umlauf befindet, praktisch gleich ist mit dem Volumen, das in dem Reaktor von dem Trägerpolymer freigelassen wird.

9. Verfahren gemäß einem der vorhergehenden Ansprüche, dadurch gekennzeichnet, daß der Reaktor (10) wärmeisoliert ist.

10. Apparatur zur Synthese von Makromolekülen wie Peptiden, Polynucleotiden oder Oligosacchariden in heterogener Phase, die einen röhrenförmigen Reaktor (10) mit einem festen Träger (12) wie einem Polymer enthält und Vorrichtungen (30, 32, 44) zur nacheinanderfolgenden Dosierung, Zuführung und Entleerung von Reaktionsflüssigkeiten und Wasch- und Spülflüssigkeiten für den Reaktor und den festen Träger, dadurch gekennzeichnet, daß der röhrenförmige Reaktor (10) an seinenäußersten Enden durch Filter (14) verschlossen ist, die den festen Träger zurückhalten und die Flüssigkeiten hindurchlaufen lassen, und dadurch, daß die Apparatur Vorrichtungen zum wiederholten Umlauf der Reaktionsflüssigkeit in abwechselnder Richtung in dem Reaktor und durch die vorgenannten Filter enthält und Vorrichtungen (36) zur Detektion der Temperaturschwankungen der Reaktionsflüssigkeit, die mit ihren Durchläufen durch den Reaktor und außerhalb des Reaktors verbunden sind.

11. Apparatur gemäß Anspruch 10, dadurch gekennzeichnet, daß die Strömung der Reaktionsflüssigkeit in abwechselnder Richtung durch den Reaktor (10) periodisch erfolgt.

12. Apparatur gemäß Anspruch 10 oder 11, dadurch gekennzeichnet, daß der Reaktor (10) beweglich montiert ist zur geradlinigen vertikalen Bewegung in abwechselnder Richtung in einen umschlossenen Raum (16), der Vorrichtungen zur Zufuhr und Entleerung der vorgenannten Flüssigkeit enthält, und daß der Reaktor mit motorbetriebenen Vorrichtungen (28) zur Verschiebung verbunden ist.

13. Apparatur gemäß Anspruch 12, dadurch gekennzeichnet, daß der Reaktor (10) so montiert ist, daß er in dem umschlossenen Raum in einer wasserdichten Führung gleitet.

14. Apparatur gemäß Anspruch 12 oder 13, dadurch gekennzeichnet, daß der Reaktor an seinen äußersten Enden mit wasserdichten Verbindungen (20) versehen ist, die mit einer zylindrischen Wand des umschlossenen Raums (16) in Verbindung stehen und zwischen sich eine Gasschicht umschließen, zum Beispiel aus Luft, die die Wärmeisolierung bildet.

15. Apparatur gemäß einem der Ansprüche 12 bis 14, dadurch gekennzeichnet, daß in dem umschlossenen Raum (16) eine konstante Temperatur aufrechterhalten wird, zum Beispiel durch Umlauf einer wärmeregulierten Flüssigkeit.

16. Apparatur gemäß einem der Ansprüche 10 bis 15, dadurch gekennzeichnet, daß die Vorrichtungen zur Temperaturdetektion wenigstens eine Temperatorsonde enthalten wie zum Beispiel einen Thermowiderstand, die in Kontakt mit der Reaktionsflüssigkeit an einem Punkt angebracht wird, der sich in der Nähe eines der vorgenannten Filter (14) befindet.

17. Apparatur gemäß Anspruch 16, dadurch gekennzeichnet, daß sich die Temperatursonde (36) außerhalb des Reaktors befindet und mit letzterem verbunden ist.

18. Apparatur gemäß Anspruch 16 oder 17, dadurch gekennzeichnet, daß die Temperatursonde (36) in einem Durchgang (40, 42) plaziert ist, der die Reaktionsflüssigkeit zum entsprechenden äußersten Ende des Reaktors führt.

19. Apparatur gemäß einem der Ansprüche 16 bis 18, dadurch gekennzeichnet, daß besagte Temperatursonde (36) mit einem Eingang zu einem Informationsverarbeitungssystem (38) verbunden ist, dessen Ausgänge mit Vorrichtungen (32) zur Steuerung der Dosierung, der Zufuhr und der Entleerung der Reaktionsflüssigkeiten verbunden sind.

20. Apparatur gemäß Anspruch 19, dadurch gekennzeichnet, daß ihr Funktionieren und die Aufeinanderfolge der Kupplungsreaktionen und der Reaktionen zur Entfernung von Schutzgruppen durch das System (38) zur Informationsverarbeitung gesteuert werden.

## Claims

1. Process for heterogeneous phase synthesis of macromolecules such as peptides, polynucleotides or oligosaccharides, consisting in depositing a solid support, such as a polymer, in a tubular reactor, in sequentially dosing and introducing reagent liquids into the reactor in order successively to effect desired coupling and stripping reactions, alternating with operations of washing and rinsing the reactor and the solid support, characterised in that it consists in closing the upper and lower ends of the reactor (10) with filters (14) that retain the solid support and allow the liquids to pass, then, for each reaction to be performed, in causing the corresponding reagent liquid to circulate repeatedly and alternately in the reactor (10) and through the aforementioned filters (14), and in monitoring the development of the ongoing reaction by detecting the variations in temperature of the reagent liquid associated with its passages in the reactor and outside the reactor, the end of the reaction corresponding to a substantially zero value of these variations in temperature after a progressive decrease over a predetermined period of time.

2. Process according to claim 1, characterised in that the alternating circulation of the liquid in the reactor (10) is periodic.

3. Process according to claim 1 or 2, characterised in that the alternating circulation of the liquid in the reactor (10) is vertical.

4. Process according to claim 1, 2 or 3, characterised in that it consists in displacing the reactor (10) in an alternating vertical rectilinear motion in a vessel (16) containing the reagent liquid.

5. Process according to one of claims 2 to 4, characterised in that it consists in continuously detecting the temperature of the reagent liquid at a point located in the immediate vicinity of a filter (14) of the reactor.

6. Process according to both of claims 4 and 5, characterised in that the point for detection of the temperature is linked to the reactor (10) and monitors it in its alternating motion.

7. Process according to claim 5 or 6, characterised in that the temperature detection point is located outside the reactor (10), in a passage (40, 42) for guiding the reagent liquid to the corresponding end of the reactor.

8. Process according to one of claims 4 to 7, characterised in that the volume of reagent liquid circulating in the reactor during each displacement thereof in one direction and in the other is substantially equal to the volume left free in the reactor by the support polymer.

9. Process according to one of the preceding claims, characterised in that the reactor (10) is thermally insulated.

10. Apparatus for heterogeneous phase synthesis of macromolecules such as peptides, polynucleotides or oligosaccharides, comprising a tubular reactor (10) containing a solid support (12), such as a polymer, and means (30, 32, 44) for sequential dosage, supply and evacuation of reagent liquids and liquids for washing and rinsing the reactor and the solid support, characterised in that the tubular reactor (10) is closed at its ends with filters (14) that retain the solid support and allow the liquids to pass, and in that the apparatus comprises means for causing the reagent liquid to circulate repeatedly and alternately in the reactor and through the aforementioned filters, and means (36) for detecting variations in temperature of the reagent liquid associated with its passages in the reactor (10) and outside the reactor.

11. Apparatus according to claim 10, characterised in that the circulation of the reagent liquid in the reactor (10) is periodic.

12. Apparatus according to claim 10 or 11, characterised in that the reactor (10) is mounted movably for alternating vertical rectilinear motion in a vessel (16) comprising means for supplying and evacuating the aforementioned liquid, and is connected to motor means (28) for displacement.

13. Apparatus according to claim 12, characterised in that the reactor (10) is mounted to slide in a sealed manner in the vessel (16).

14. Apparatus according to claim 12 or 13, characterised in that the reactor includes at its ends sealing gaskets (20), cooperating with a cylindrical wall of the vessel (16) and between them trapping a layer of gas, for example air, forming a thermal insulation.

15. Apparatus according to one of claims 12 to 14, characterised in that the vessel (16) is kept at constant temperature, for example by circulation of a thermoregulated liquid.

16. Apparatus according to one of claims 10 to 15, characterised in that the temperature detection means comprise at least one temperature probe, such as a thermistor, for example, placed in contact with the reagent liquid at a point located in the vicinity of one of the aforementioned filters (14).

17. Apparatus according to claim 16, characterised in that the temperature probe (36) is on the outside of the reactor and is connected to it.

18. Apparatus according to claim 16 or 17, characterised in that the temperature probe (36) is placed in a passage (40, 42) that conducts the reagent liquid to the corresponding end of the reactor.

19. Apparatus according to one of claims 16 to 18, characterised in that said temperature probe (36) is connected to an input of an information processing system (38), the outputs of which are connected to means (32) for controlling dosage, supply and evacuation of the reagent liquids.

20. Apparatus according to claim 19, characterised in that its function and the succession of the coupling and stripping reactions are controlled by the information processing system (38).
